# EUROPEAN PATENT APPLICATION

(11) **EP 3 293 263 A1**
(43) Date of publication of application: **14.03.2018**
(21) Application number: 17189614.5
(22) Date of filing: 06.09.2017
(51) Int. Cl.: C12N 15/10, C12N 15/11, C12N 15/63, C12N 15/90

(54) **INDUCIBLE GENE EXPRESSION BY LINEAR AT-RICH DNA FRAGMENTS IN MAMMALIAN CELLS AND METHODS RELATING THERETO**

(30) Priority: 07.09.2016 EP 16001951
(71) Applicant: Steinbeis Innovation gGmbH, 70174 Stuttgart (DE)
(72) Inventor: FREY, Manfred, 68259 Mannheim (DE); BERNHARD, Kevin, 68169 Mannheim (DE)
(74) Representative: Lahrtz, Fritz

(57) **Abstract**

The present invention relates to a method for providing cell lines expressing at least two different recombinant proteins of interest, comprising the steps of transfecting mammalian cells with at least one linear DNA fragment comprising or consisting of an AT-rich nucleotide sequence and, in parallel, with at least one plasmid encoding the at least two different recombinant proteins of interest. The invention further relates to methods for providing cell based assays for analyzing the function of a protein complex of interest using these cell lines and mammalian cell lines characterized by a linear DNA fragment comprising or consisting of an AT-rich nucleotide sequence.

## Description

The invention relates to methods for providing mammalian cell lines expressing at least two different recombinant proteins of interest using an inducible expression system. The invention further relates to cell based assays using these mammalian cell lines. The mammalian cell lines of the present invention harbor an inducible expression system which is modulated by an AT-rich DNA element which interacts with the complex of a transcriptional activator and its corresponding operator site in form of a transacting factor. The at least two different recombinant proteins of interest expressed by the mammalian cell lines of the present invention are further characterized in that one of them represents a transcriptional activator whereby the expression of said transcriptional activator is regulated by an inducible expression system consisting of an operator site specific for this specific transcriptional activator. Due to a conformational change of the transcription factor domain, the transcriptional activator binds to its operator site resulting in a self-amplification of its own expression and the expression of at least one protein of interest at the same time.

Both in basic research as well as for commercial applications eukaryotic and prokaryotic cells are commonly used for the expression of nucleotide sequences encoding a product of interest (e.g. a polypeptide or a functional RNA). For the synthesis of a product of interest in a host cell, a gene encoding said product is introduced into host cells, e.g. by transfection or transformation. The cells are then grown under suitable conditions to express the product of interest. This cultivation step often includes the use of a selectable marker gene and/or the use of reporter gene(s) which are introduced into the host cells together with the nucleotide sequence encoding the product of interest in order to select the cells which harbour the product of interest. After the cultivation step, the recombinant protein or the recombinant protein complex of interest may be analysed and visualised by means of optical and/or functional readout.

Continuous high level expression of an immunoglobulin in CHO cells is known in the art (1). Here, high-level expression was achieved by adding a chicken MAR element both *in cis* (i.e. on the transgene expression vector) and *in trans* (i.e. by co-transfection on a separate plasmid).

Inducible high level expression of a reporter protein luciferase was achieved by the co-transfection of four plasmids (2). The inducible expression was enhanced by co-transfection of a chicken MAR containing plasmid and three plasmids coding for a chimeric repressor, a conditional trans-activator and one transgene (3). For the development of cell based test systems, sensor cell lines are used which have to express different recombinant proteins at the same time at high expression levels. The expression of various recombinant protein complexes, like for example ion channel proteins, is often associated with a significant stress for this cell line which often results in dying of the cells. Therefore, sophisticated sensor cells like ion channel expressing cell lines have to be constructed by using inducible expression systems allowing the high-level expression of several different proteins. Described inducible expression systems require a constitutively expressed transcription factor fusion protein (transcriptional activator or repressor) which is excessively resource demanding for the cell especially in the case of the simultaneous high level expression of several different proteins (3 to 5). Such inducible expression systems based on different origin and effector molecules are described as transcriptional switches.

Mammalian transgene control switches have been developed where the switches are controlled by either antibiotics (6), hormones and hormone analogs (7), quorum sensing substances (6), amino acids (6), vitamins (8), acetaldehyde (6), food additives (6), temperature (6), or blue light (6). The development of a recombinant cell line expressing different proteins at a well-defined stable high expression level after induction is an extremely difficult task. Therefore, a cumbersome screening is required to find one positive clone with sufficient expression levels and stability of the necessary protein components used for the development of a useful sensor cell line. If a new sensor cell line and screening parameters for the sub-cloning have to be developed, a good yield of positive cell clones expressing all genes of interest at the same time at high expression level is crucial for a successful development.

Hence, there is always a need for improved inducible cell based expression systems for the simultaneous expression of more than one recombinant protein in one host cell.

It has surprisingly been found that the limitations known in the art can be overcome by the co-transfection of AT-rich DNA elements (1). That is, AT-rich DNA elements are known in the art to improve the yield of positive cell populations surviving the selection process and to improve the inducible protein expression level and the stability of the cell lines.

In the context of the present invention, it has surprisingly been found that a process of self-amplification of a transcriptional activator can be induced within a cell in the absence of any chimeric repressor or transactivator gene expression under the control of a continuous promotor within the cell. The self-amplification is mediated by the transfection of a transcriptional activator which expression is self-induced due to the regulation of the expression by the same gene switch, wherein the transcriptional activator contains a transcription factor domain modulated by an AT-rich DNA element, and wherein the AT-rich DNA element interacts with the complex of the transcriptional activator and its corresponding operator site in form of a transacting factor.

Hence, due to the surprising findings of the present invention, it is possible to use a promotor with at least one operator site responsive for binding of the transcription factor domain which interacts with the AT-rich DNA element. That is, during the "off-state", surprisingly low amounts of the transcriptional activator are expressed, thereby allowing its self-amplification. After a conformational switch of the transcription factor domain triggered by substrate binding the transcriptional activator binds to its operon and induces the expression of itself and all gene of interests being under the expression control of the same gene switch.

The present invention is directed to mammalian cells harboring transcription factors and respective promotors, enabling an inducible expression, whose functionality is significantly improved due to the modulation by AT-rich DNA elements. These AT-rich DNA elements modulate the interaction of promotor and respective transcription factor *in trans,* meaning that these elements are not part of the DNA sequence harboring the gene of interest. Rather, the AT-rich DNA element integrates together with the gene of interest after co-transfection of both DNAs into the host cell chromatin. The invention is further directed to a method for developing mammalian cell lines expressing several recombinant proteins at once after induction of their expression. The invention further relates to the combination of two different transcriptional switches which are both modulated by the same AT-rich DNA element.

In one aspect, the present invention relates to a method for providing a cell line expressing at least two different recombinant proteins, comprising the steps of
a) providing mammalian cells in cell culture;
b) providing at least one plasmid, wherein the at least one plasmid contains a DNA sequence encoding for a transcriptional activator under the control of at least one promotor region operably linked to at least one operator site (OP), wherein the operator side (OP) is responsive for binding the transcription factor domain of the transcriptional activator;
c) transfecting the mammalian cells with at least one linear DNA fragment comprising or consisting of an AT-rich nucleotide sequence and, in parallel, with the at least one plasmid of step b), characterized in that the plasmid of step b) further contains a DNA sequence encoding for at least one recombinant protein of interest, wherein the expression of the at least one recombinant protein of interest is under the control of the same gene switch as the transcriptional activator;
d) inducing the concomitant self-amplification of the transcriptional activator and the expression of the at least one recombinant protein of interest by incubating the mammalian cells under conditions of a transcriptional switch;
e) monitoring the level of expression of the at least one recombinant protein of interest by means of visual inspection or by means of a functional readout.

In the context of the present invention, the term "expression of a recombinant protein" or "expressing a recombinant protein" may either refer to the expression of a transcriptional activator or to the expression of a different recombinant protein of interest, or to both. That is, providing a cell line expressing at least two different recombinant proteins according to the present invention means that both a transcriptional activator and, in addition, at least one different recombinant protein of interest is expressed within one cell. In a preferred embodiment, a transcriptional activator and at least two different recombinant proteins of interest are expressed within one cell.

A transcription factor (TF) according to the present invention may, in principle, be any protein which influences the transcription activity of an operator site (OP) containing promotor sequences in response to an interaction with a respective regulating compound (RC). In the context of a present invention, a regulating compound may be any low molecular weight compound which interacts with a transcription factor (TF) and thereby influences the binding affinity of the transcription factor to a responsive promotor harboring at least on operator site (OP) to which the transcription factor can bind. The regulating compound (RC) can be selected, as non-limited examples, from antibiotics like doxycycline or coumermycin.

The transcription factor may comprise proteins identical to, derived from or related to proteins naturally encoded by prokaryotes or eukaryotes. The term proteins "derived from" naturally occurring proteins generally means in this context that the transcription factor comprises protein domains that contain amino acid substitutions, but remain at least 70% identical to the naturally occurring proteins at the amino acid level.

The operator site (OP) consists of at least one operator sequence (OS) derived from a naturally occurring DNA sequence or a DNA sequence derived from a naturally occurring sequence. The operator sequence (OS) may consist of a naturally occurring DNA sequence or a DNA sequence derived from a naturally occurring sequence which can be bound by a TF constitutively or in RC dependent way. In the context of the present invention, the term "by an OS derived from a naturally occurring DNA sequence" is meant to define that the DNA sequence of the OS contains base changes compared to the naturally occurring polynucleotide still binding to the TF either constitutively or in an RC-dependent way.

The term "plasmid" (also referred to herein as a "vector" or "nucleic acid expression construct") generally refers to any kind of nucleic acid molecule that can be expressed in a cell. In particular, the plasmid of the invention can be any plasmid or vector suitable for simultaneously expressing a least two recombinant proteins of interest in a host cell of interest, such as dividing or non-dividing cells, including, but not limited to, plasmids or vectors designed for transient or stable transfection. Preferably, the plasmid of the invention is an expression construct suitable for stable cell transfection. The terms "plasmid" or "vector" include all nucleic acid constructs that are suitable for introducing or transferring a nucleic acid sequence encoding a gene of interest into a cell. Such constructs are well known in the art, and can be commercially purchased from diverse suppliers including, e.g., Promega™ (Madison, WI, USA), Qiagen® (Hilden, Germany), or Invitrogen™ (Carlsbad, CA, USA). The plasmid or vector of the present invention may comprise the DNA of a transmissible agent into which foreign DNA can be inserted. A common way to insert one segment of DNA into another segment of DNA involves the use of enzymes called restriction enzymes that cleave DNA at specific sites (e.g. palindrome sequences) called restriction sites. A "cassette" refers to a DNA coding sequence or a segment of DNA that encodes an expression product that can be inserted into a plasmid or vector at defined restriction sites. The term "transfection" as used herein refers to all kind of methods known in the art that are suitable for introducing a nucleic acid sequence encoding a gene of interest into a host cell, in particular into a mammalian cell. Such methods are well known to the person skilled in the art and are, e.g., described in Sambrook et al., 2000 (Molecular Cloning: A laboratory manual, Third Edition).

In a preferred embodiment, the method for providing a cell line includes the co-transfection of at least two plasmids in step c) along with the at least one DNA fragment comprising or consisting of the AT-rich nucleotide sequence. If two plasmids are transfected in step c), preferably each of the two plasmids contains a DNA sequence encoding for at least one recombinant protein of interest and, optionally, a DNA sequence encoding for a transcriptional activator. The expression of the at least two recombinant proteins of interest may be under the control of same gene switch, or alternatively, under the control of different gene switches wherein in the latter scenario, each of the plasmids encodes at least one recombinant protein of interest together with one transcriptional activator. Preferably, two different transcriptional switches are combined, wherein both transcriptional switches are modulated by the same AT-rich DNA element.

In yet another preferred embodiment, at least three individual plasmids, more preferably at least four individual plasmids encoding for at least three, four, five, six or more different recombinant proteins of interest are co-transfected in addition to the DNA sequence encoding the transcriptional activator and in addition to the at least one DNA fragment comprising or consisting of the AT-rich nucleotide sequence. Alternatively preferred is that the plasmid transfected in step c) encodes at least three or four different recombinant proteins of interest. Plasmids encoding at least three or more different recombinant proteins at once are designed as such that diverse open reading frames are inserted into one vector under the control of appropriate promoter sequences, including, but not limited to bidirectional promotor sequences and eukaryotic internal ribosome entry sites.

Alternatively preferred is that two individual plasmids are transfected, wherein each plasmid encodes at least one recombinant protein of interest in addition to at least one transcriptional activator. Preferably, the two individual plasmids transfected in step c) each encode at least two, at least three or four different recombinant proteins of interest. Equally preferred is that the two individual plasmids, taken together, encode 4, 5, 6, 7, 8 or more different recombinant proteins at once in one host cell, thereby allowing for the simultaneous expression and assembly of large intracellular functional protein complexes constituted by 4, 5, 6, 7, 8 or more individual protein components.

The term "promoter" as used in the context of the present invention generally refers to any kind of regulatory DNA sequence operably linked to a downstream coding sequence, wherein said promoter is capable of binding RNA polymerase and initiating transcription of the encoded open reading frame in a cell, thereby driving the expression of said downstream coding sequence. In order to ensure expression of the recombinant proteins in the context of the present invention, the plasmid of the invention comprises one or more non-endogenous promoter sequence(s), including, but not limited to bidirectional promoter sequences, that are operably linked to the nucleic acid sequences encoding the at least one or more proteins of interest and which are suitable for driving the expression of one or more recombinant protein(s) in a host cell, preferably in a mammalian host cell. The non-endogenous promoter sequence of the present invention can be any kind of promoter sequence known to the person skilled in the art, including, but not limited to, inducible promoters, cell cycle-specific promoters, cell type-specific promoters and eukaryotic internal ribosome entry site elements containing cryptic promoter activity.

In a preferred embodiment of the present invention, the at least one promotor region operably linked to the at least one operator site (OP) is a bidirectional promotor comprising two promotor regions.

The plasmid (also referred to as a nucleic acid expression construct or vector) of the present invention might further comprise all other sorts of nucleic acid sequences, including, but not limited to, polyadenylation signals, splice donor and splice acceptor signals, intervening sequences, transcriptional enhancer sequences, translational enhancer sequences, drug resistance gene(s) or alike. Optionally, the drug resistance gene and the fluorescence reporter gene may be operably linked by an internal ribosome entry site (IRES). In particular, the plasmid of the present invention may allow for the production of either large or small amounts of stable mRNA once it is introduced into a cell of interest. The plasmid may further contain any kind of regulatory sequences that function as enhancer or silencer elements, and which might regulate and/or modulate the activity of the promoter and thereby the rate of transcription and thus expression of the proteins of interest. Additionally, the plasmid may additionally contain a strong termination codon, an insertion of transcription termination sequence and a PTIS (portable translation initiation sequence), and may be designed as to provide an adjustment of distance between promoter and cloned gene of interest.

The promotor of the invention is preferably an inducible promoter. The term "inducible promoter" as referred to herein generally means any promoter known to the person skilled in the art that regulates expression in response to a stimulus. An inducible promoter of the present invention can be bound by a transcription factor that either represses or activates gene expression. The repressor or activator protein in turn is responsible to the stimulus, such as the presence or absence of a nutrient, a protein, or some other environmental signal. Inducible promoters of the present invention may include, but are not limited to, the pTet-on/pTet-off system. Such systems are well known in the art and can be commercially purchased from, e.g, Clontech™ (USA).

The term "expressing" or "expression" as used herein generally mean allowing or causing the information in a gene or a DNA sequence to become manifest. In particular, in the context of the present invention, it means the intracellular production of the protein encoded by the nucleic acid expression construct of the present invention by activating the cellular functions involved in transcription and translation of the corresponding gene or DNA sequence. That is, a DNA sequence is expressed in or by a cell to form an "expression product" such as a protein. The simultaneous expression of the at least two proteins of interest may depend on a variety of factors, including, but not limited to, the promoter sequence which is operably linked to the DNA sequence encoding the protein of interest, the presence or absence of enhancer and/or silencer sequences or other DNA sequences which control the rate of transcription, the cell culture conditions applied to the cell carrying the respective DNA sequence including the media used for culturing the cell, and/or the number of copies of DNA sequences introduced into the cell. Successful expression of the protein may be analyzed and/or visualized by standard methods known to the person skilled in the art, including, but not limited to, Western Blot analysis, Northern Blot analysis, RNase protection assays, quantitative RT-PCR, as well as methods concerning direct or indirect fluorescence readout. Such methods are, e.g., described in Sambrook, 2000 (Molecular Cloning: A laboratory manual, Third Edition).

In particular, in the context of the present invention, the term "transfection" preferably refer to the transfection of one or more nucleic acid sequences into a host cell, preferably into mammalian host cells such as, for example, CHO cells. Transfection of DNA into mammalian cells typically involves opening transient pores in the cell plasma membrane, to allow the uptake of material. In addition to electroporation, transfection can be carried out using calcium phosphate, or by mixing a cationic lipid with the material to produce liposomes, which fuse with the cell plasma membrane and deposit their cargo inside. The nucleic acid sequence(s) introduced into the host cell of the invention can be derived from any kind of source, including cells of the same genus or species as the host cell, or cells of a different genus or species.

In the context of the present invention, the mammalian cells are transfected with at least one plasmid of interest and, in addition, with at least one AT- rich DNA element which does not form part of the transfected plasmid. Hence, the methods of the present invention are defined by a co-transfection of a linear DNA fragment revealing AT-rich DNA sequences and the plasmid(s) of interest.

Preferably, the mammalian cells are transfected with at least one plasmid containing a DNA sequence encoding for a transcriptional activator under the control of a gene switch allowing its self-amplification.

In the context of the present invention, the plasmid or nucleic acid expression construct is preferably introduced into the mammalian cell by means of stable transfection.

"Stable transfection" as referred to herein means any kind of stable gene transfer in which a nucleic acid encoding a gene of interest is introduced into a target cell as such that it is stably integrated into the genome of said cell. Stable gene transfer can be carried out by diverse standard methods known to the person skilled in the art including, but not limited to, the use of viral vectors for expression in both dividing and non-dividing cells. Stable gene transfer is mostly accompanied by the co-introduction of a selection gene that confers resistance toward a certain substance, e.g., a certain toxin. Examples of these substances include Geneticin, also known as G418, which is a toxin that can be neutralized by the product of the neomycin resistant gene. If the toxin, towards which the co-transfected gene offers resistance, is then added to the cell culture, only those few cells with the foreign genes inserted into their genome will be able to proliferate, while other cells will die. After applying this selective selection procedure for some time, only the cells with a stable transfection remain and can be cultivated further.

The term "monitoring" as used herein generally refers to the visual inspection of a cell or of a population of cells. In particular, monitoring according to the method of the present invention means the visualization of the expression of the at least two recombinant proteins of interest by the help of reporter proteins. Alternatively, the levels of expression may also be analyzed by means of functional readout assays, including, but not limited to, for example, patch clamp analysis or microelectrode assays. Functional assays in this respect are known to the skilled person. That is, the term "functional readout" as used in the context of the present invention generally means any kind of assay which is suitable for analyzing and/or evaluating the function of a recombinantly expressed protein or of a recombinantly expressed protein complex of interest within a cell. The intracellular function of the recombinant protein of interest or of the recombinant protein complex of interest may be of any kind, such as enzymatic function(s), metabolic function(s), catalytic function(s), structural function(s), internal transport function(s), functions associated with intracellular uptake or membrane assembly, conductive or funneling function(s) or alike.

In certain embodiments, the inducible expression of the at least two reporter proteins within the mammalian host cell may be continuously monitored for a certain period of time after transfection and induction. This period of time may include several hours, preferably a time period of at least 14 hours, but may also refer to a time period of several days or more. During this time, the cells are expected to undergo at least one or more rounds of cell cycles. Furthermore, the cells may also undergo multiple or even an unlimited number of cell cycles. The expression of the at least one or two reporter protein(s) of interest can be monitored by visual inspection of the cell(s) at defined time points over a certain period of time, preferably by means of quantitative fluorescence readout, in which the cell(s) preferably undergo at least one round of cell cycle. This period of time may vary from cell type to cell type and thus depends on the precise type of cell or cell population analyzed.

In a preferred embodiment, the method of the invention is characterized in that the visual inspection is by means of fluorescent readout.

Preferably, the visual inspection is by means of quantitative fluorescent readout.

Fluorescent readout according to the present invention generally includes the visualisation of one or more recombinant protein(s) of interest by means of fluorescence detection, such as, for example, the visualisation of a fluorescent reporter protein. Preferably, the fluorescent reporter protein of the present invention may be a green fluorescence protein (GFP) from *Aequorea victoria* including any known functional analogues thereof in which the amino acid sequence of wild type GFP has been altered by amino acid deletion, addition, or substitution. Suitable GFP analogues for use in the present invention include, but are not limited to, EGFP (enhanced green fluorescence protein), EYFP (enhanced yellow fluorescence protein), and ECFP (enhanced cyan fluorescence protein). These fluorescence reporter proteins are well known in the art, and can, e.g., be commercially purchased from Clontech™, USA. Other fluorescence reporter proteins may include, but are not limited to, *Aequorea coerulescence* jellyfish fluorescent protein (AcGPF1), DsRed, HcRed, AsRed, ZsGreen, ZsYellow, AmCyan fluorescent protein (BD Clontech, USA), Renilla GFP (Stratagene, USA), monomeric Kusabira-Orange (mKO1 and mKO2) and monomeric Azami-Green (mAG) (MBL Medical & Biological Laboratories Co., Ltd.), mCherry, mKate, mKatushka, TurboRFP and TagRFP, and any functional fragment or derivative thereof.

In a particularly preferred embodiment, the fluorescent reporter protein of the present invention is EGFP (enhanced green fluorescence protein) or mCherry, or any functional fragment or derivative thereof.

The term "fluorescence readout" as used herein generally refers to all sorts of imaging methods known in the art that are suitable to visualize, detect, analyze and/or quantify the expression of the fusion protein encoded by the nucleic acid expression construct of the present invention within a cell. In particular, fluorescence analysis according to the present invention includes, but is not limited to, all known methods of deconvolution and confocal fluorescence microscopy, in particular methods concerning the *in vivo* imaging of the fusion protein such as, e.g., time-lapse microscopy and/or live cell imaging as well as methods concerning multiple-fluorescent-probe microscopy. Fluorescence analysis according to the present invention also includes all known methods of stimulated emission depletion (STED) microscopy. The visualization of fluorescence proteins in subcellular structures by means of STED microscopy is a technique well known to the person skilled in the art.

Preferably, the concomitant self-amplification of the transcriptional activator and the expression of the additional recombinant protein(s) of interest in step d) is induced by incubating the mammalians cells under conditions of the same transcriptional switch. Alternatively preferred is that the concomitant self-amplification of the transcriptional activator and the expression of the additional recombinant protein(s) of interest in step d) is induced by incubating the mammalians cells under conditions of different transcriptional switches.

In the context of the present invention, the term "transcriptional switch" generally refers to any mammalian transgene control switch setup which includes the molecular interaction of a particular transcription factor with a corresponding DNA sequence know as an operator site (OP). Preferably, the transcriptional switch of the present invention means an inducible concomitant expression of at least two different recombinant proteins of interest, wherein the mammalian cells are incubated under conditions of the so called "dox-off system, The dox-off system of the invention is preferably characterized by the interaction of the Tn10-encoded Tet repressor protein (TetR) and a corresponding operator site (OP) like, for example, the Tn10-specified tetracycline-resistance operon of E. coli (9).

Particular pairs of TF and OP binding partner are, for example, the Tn10-encoded Tet repressor protein (TetR) that regulates the expression of tetracycline resistant genes in gram negative bacteria in a tetracycline dependent fashion. In the absence of the RC tetracycline, a TetR dimer binds to the OS, consisting of tandem tetracycline OS sequences OS1 and OS2, and inhibits expression of the tetracycline resistant gene. When the RC enters the cell and binds to TetR, the affinity for OS is reduced and TetR dissociates from OP allowing expression of the tetracycline resistant gene. The TF may be an assembly of different protein domains, which may comprise protein domains which either activate or repress transcription of promotors functionally linked to OP. Activation domains useful for the present invention are, for example, the Herpes simplex VP16 domain or minimal versions thereof. For the purpose of the invention a transcriptional switch based on a TetR fused to the Herpes simplex VP16 domain and an appropriate OP is called "dox-off system".

In another preferred embodiment, the inducible concomitant self-amplification and expression of the at least two different recombinant proteins involves incubating the mammalian cells under conditions of the coumermycin-on system, wherein the coumermycin-on system is characterized by the interaction of a fusion protein composed of the N-terminal domain of the λ repressor fused to the bacterial DNA gyrase B subunit followed by the transcription activation domain p65 NF-κB and a corresponding operator site (OP).

Another pairs of TF and OP binding partner are, for example, the fusion protein comprising a N-terminal domain of the λ repressor (the cl gene product of bacteriophage λ) fused to the bacterial DNA gyrase B subunit followed by the transcription activation domain p65 NF-κB described in (5). After adding of the RC coumermycin into the medium, RC binds to its TF and expression of the gene functionally linked to the OS is induced. For the purpose of the invention a gene switch based on a fusion protein comprising a N-terminal domain of the λ repressor fused to the bacterial DNA gyrase B subunit followed by the transcription activation domain p65 NF-κB and an appropriate OP is called "coumermycin-on system".

In the context of the present invention, the term "fusion protein" generally means any kind of protein which is designed by a reporter gene and a gene of interest. Reporter genes can be used to assay for the expression of the gene of interest, which may produce a protein that has little obvious or immediate effect on the cell culture or the organism. In these cases, the reporter is directly attached to the gene of interest to create a gene fusion. That is, the two genes are under the same promoter and are transcribed into a single messenger RNA molecule. The mRNA is then translated into protein. In these cases, it is thus important that both proteins are able to properly fold into their active conformations and interact with their substrates despite being fused. In building the DNA construct, a segment of DNA coding for a flexible polypeptide linker region is usually included so that the reporter and the gene product will only minimally interfere with one another.

In a preferred embodiment, the at least one linear DNA fragment comprising or consisting of an AT-rich nucleotide sequence which is to be co-transfected with the plasmid(s) in the context of the present invention has a length of between 3500 and 2700 nucleotides. Preferably, the linear DNA fragment of the invention has a length of between 3300 and 2800 nucleotides, more preferably a length of between 3280 and 2900 nucleotides, or any combination of lengths of the aforesaid.

In yet another preferred embodiment, the at least one linear DNA fragment comprising or consisting of an AT-rich nucleotide sequences comprises or is composed of a sequence corresponding to the AT rich MII region of intron 13 of human topoisomerase I.

Preferably, the sequence corresponding to the AT rich MII region of intron 13 of human topoisomerase I comprises or consists of SEQ ID NO. 3.

Preferably, the at least one linear DNA fragment comprising or consisting of an AT-rich nucleotide sequence has a sequence according to SEQ ID No. 1, SEQ ID No. 2 or SEQ ID No. 3, or any combination thereof. More preferably, the linear AT-rich DNA fragment has the sequence of SEQ ID No. 1. The co-transfection of the linear DNA fragment according to the present invention together with the at least one plasmid is further exemplified in Example 1.

Preferably, the at least one linear DNA fragment comprising or consisting of an AT-rich nucleotide sequence is composed of a nucleotide sequence according to SEQ ID No. 1, SEQ ID No. 2 or SEQ ID No. 3, or any combination thereof. More preferably, the linear AT-rich DNA fragment of the invention has the sequence of SEQ ID No. 1. The co-transfection of the linear AT-rich DNA fragment together with a plasmid encoding for at least two different recombinant proteins of interest according to the present invention is further exemplified in Example 1.

In another preferred embodiment, the at least one linear DNA fragment comprising or consisting of an AT-rich nucleotide sequence is co-transfected with the plasmid(s) of the present invention at a molar ratio of between 0.1 (AT-rich DNA fragment) to 8 (plasmid), preferably a molar ratio of between 0.2 (AT-rich DNA fragment) to 6 (plasmid), more preferably a molar ration of between 0.5 (AT-rich DNA fragment) to 4 (plasmid).

In another aspect, the present invention relates to a method for providing a cell based assay for analyzing the function of a protein complex of interest, comprising the steps of
a) providing mammalian cells in cell culture, wherein the cells are derived from a cell line obtained by the method of the present invention; and
b) subjecting the mammalian cells to conditions of a cell assay, wherein the cell assay is characterized by
   i) measuring the intracellular concentration of bicarbonate; or
   ii) measuring the intracellular concentration of calcium; or
   iii) measuring the activity of ion channels; or
   iv) measuring the activity of the ion channel Cav1.2; or
   v) measuring intracellular cyclooxygenase activity; or
   vi) measuring the activity of G-protein coupled receptors (GPCRs); or
   vii) measuring the activity and/or the inhibition of the G-protein coupled beta-adrenergic receptor.

Cell based assays for analyzing the function of a protein complex of interest according to the present invention including the conditions to which the mammalian cells are subjected to are, for example, detailed and exemplified in Example 2, 3, 5, 6, and 7 of the present invention. It is well known to the skilled person that the conditions of the respective cell assay may vary dependent of the particular mammalian cell line employed as well as dependent on the particular measurement carried out. The conditions of the cell assays for measuring the function of a particular protein complex of interest include, but are not limited to, the diverse embodiments as described in the context of the present invention. The measurement of enzymatic function(s), the measurement of intracellular ion concentration(s) of any kind, and the measurement of the activity of ion channels are subject to standard methods known to the skilled person.

In a further aspect, the present invention is directed to a mammalian cell line, wherein the mammalian cell line is characterized by
i) the presence of a transcription factor (TF), wherein the transcription factor binds to an operator site (OP) containing promotor sequence in response to the presence of at least one compound which is to be added to the cell culture medium, or, alternatively, in response to the absence of at least one compound which is to be removed from the cell culture medium;
ii) the presence of a promotor sequence, or a fragment thereof, operatively linked to the operator site (OP), wherein the operator site is specific for binding of the transcription factor (TF);
iii) the presence of a DNA fragment comprising or consisting of an AT-rich nucleotide sequence, wherein the DNA fragment results from the integration of a linear DNA fragment.

Preferably, the mammalian cell line is characterized in that the linear DNA fragment comprising or consisting of an AT-rich nucleotide sequence allows for the self-amplification of the transcriptional activator after its integration into the cell's genome without the need of a constitutively expressed chimeric repressor or transactivator.

The term "operably linked" as used herein means that the gene elements are arranged as such that they function in concert for their intended purposes, e.g. in that transcription is initiated by the promoter and proceeds through the DNA sequence encoding at least one protein of interest. That is, RNA polymerase transcribes the sequence encoding the protein of interest into mRNA, which in then translated into a protein.

In the context of the present invention, the linear DNA fragment comprising or consisting of an AT-rich nucleotide sequence is preferably capable of modulating the interaction between the transcription factor and the promotor sequence, preferably after its integration in the chromatin of the host DNA. Preferably, the AT-rich nucleotide sequence is capable of modulating the transcriptional machinery of the cell and the inducible promotor in a way that allows the expression of a transcriptional activator at low amounts which enable a self-amplification of the transcriptional activator without the need of a constitutively expressed chimeric repressor or transactivator which expression is regulated by a constitutive promotor. In addition, in the context of the present invention the linear DNA fragment comprising or consisting of an AT-rich nucleotide sequence is preferably capable to enhance or strengthen the molecular interaction between the transcription factor to its corresponding operator side after integration in the chromatin of the host DNA.

"Capable of modulating" preferably means that the molecular interaction between the transcription factor and its cognitive binding site, i.e. the binding of the transcription factor to its corresponding operator site (OP), is enhanced or strengthened or both. More preferably, the linear DNA fragment of the invention comprising or consisting of an AT-rich nucleotide sequence is capable of modulating the interaction of the transcription factor with the operator site (OP) after its integration in the host chromatin.

Equally preferred is that the linear DNA fragment comprising or consisting of an AT-rich nucleotide sequence is capable of enhancing expression of a co-transfected gene of interest after its integration.

The term "presence of a linear DNA fragment" is to be understood as such that the linear DNA fragment has been stably transfected and introduced into the mammalian cell line of the invention, preferably by integrating into the chromatin of the cell nucleus.

In a preferred embodiment of the invention, the linear DNA fragment comprising or consisting of an AT-rich nucleotide sequence corresponds to the sequence of the AT-rich MII region of intron 13 of human topoisomerase I. Preferably, the linear DNA fragment has a sequence which is identical to sequence of the AT-rich MII region of intron 13 of human topoisomerase I (SEQ ID NO: 3).

For the purpose of the invention, the AT-DNA comprises a DNA element modulating the interaction of the TF with the corresponding OP. It is to be understood that said AT-DNA is capable of enhancing expression of a co-transfected gene of interest. The AT-DNA comprises a sequence which is related to the AT rich MII region of intron 13 of human topoisomerasel I (10). By "a sequence which corresponds to the AT rich MII region of intron 13 of human topoisomerase I" is meant, for the purpose of the invention, that the polynucleotide sequence hybridizes to the naturally occurring polynucleotide sequence (11) under at least low stringency conditions, more preferably moderate stringency conditions and most preferably high stringency conditions.

Preferably, the AT-rich DNA fragment comprises or consists of the nucleic acid sequence of SEQ ID NO. 1 (corresponding to or comprising GenBank: GM773871.1). More preferably, the AT-rich DNA fragment comprises or consists of the nucleic acid sequence of SEQ ID NO. 2 (corresponding to or comprising GenBank: GM739546.1 comprising the AT rich MII region of intron 13 of human topoisomerase I (10, 11). Most preferably, the AT-DNA comprises or consists of the the nucleic acid sequence of SEQ ID NO. 3 (corresponding to or comprising GenBank: GM739547.1)

More preferably, the linear AT-rich DNA fragment of the invention comprises or consists of a sequence according to SEQ ID No. 1, SEQ ID No. 2 or SEQ ID No. 3, or any combination thereof.

In another preferred embodiment, the mammalian cell line of the invention is a cell line that stably expresses
a) at least two recombinant proteins, wherein the at least two recombinant proteins enable the measurement of intracellular bicarbonate concentration changes;
b) at least two recombinant proteins, wherein the at least two recombinant proteins enable the measurement of intracellular calcium concentration changes;
c) at least two recombinant proteins, wherein the at least two recombinant proteins enable the measurement of ion channel activity;
d) at least two recombinant proteins, wherein the at least two recombinant proteins enable the measurement of the Cav1.2 ion channel activity;
e) cyclooxygenase and a redox sensor protein, wherein the cyclooxygenase and the redox sensor protein enable the measurement of cyclooxygenase activity;
f) at least two recombinant proteins, wherein the at least two recombinant proteins enable the measurement of G-protein coupled receptor activity;
g) at least two recombinant proteins, wherein the at least two recombinant proteins enable the measurement of beta adrenergic receptor activity.

Mammalian cell lines according to the present invention expressing at least two recombinant proteins of interest are exemplified in Example 2, 3, 4, 5, 6, and 7 of the present invention. In this context, the term "enable" ore "enabling" is to be understood as such that the at least two recombinant proteins of interest are expressed at such levels that the function of these proteins (either enzymatic or catalytic or of any kind as exemplified in the context of the present invention), or, alternatively, the function of a protein complex which is assembled by the individually expressed protein components may be analyzed, evaluated and/or measured by standard biochemical or biophysical assays known to the skilled person.

In a preferred embodiment, the mammalian cell line of the present invention is characterized by the expression of at least three, four, five or six different recombinant proteins which enable the measurement of a particular function. Equally preferred is that the mammalian cell line expresses even more different recombinant proteins as set forth above.

In sum, the present invention is directed to mammalian cells comprising a transcription factor (TF) and a corresponding OP which may be functionally linked to promotor sequences. For the induction of the expression, the gene of interest may be fused downstream of an artificial or natural promotor or a promotor fragment containing at least one OS.

The invention is further directed to a method of using mammalian cells containing at least one TF and at least one corresponding OP binding partner and at least one gene of interest and an integrated AT-rich DNA fragment (AT-DNA) for modulating gene expression to the respective exogenous or endogenous administration of RCs. Therefore, the cells may be co-transfected with a DNA element harboring the genetic information of the TF, the OP, a gene of interest fused downstream to a promotor or a promotor fragment together with an AT-DNA localized on a separate DNA molecule. The AT-DNA is not part of the sequence harboring the transfected gene(s) and integrates together with the gene(s) after co-introduction of both separate non-covalently linked DNAs into the host cell chromatin. Thus the AT-DNA interacts with the OP-TF complex like a trans-acting factor. By co-introduction of the AT-DNA the functionality of transcription factors and respective promotors, enabling the inducible expression, is thereby significantly improved. Surprisingly, it has been found that the integrated linear DNA fragment comprising or consisting of an AT-rich nucleotide sequence is preferably capable of modulating the interaction between the transcriptional machinery of the cell and the inducible promotor in a way that allows the expression of a transcriptional activator at low amounts which enable a self-amplification of the transcriptional activator without the need of a constitutively expressed chimeric repressor or transactivator by the cell. In addition, the co-introduction of AT-DNA results in a significant improvement of the yield of positive cell clones surviving the selection pressure. This is unexpected because the AT-DNA is not covalently linked to the DNA comprising the gene of interest and the expression is switched off during the selection pressure. After the selection process the co-introduction of AT-DNA results in positive cells unexpectedly expressing their gene of interest in a stable well-defined amount and high expression level after induction of the gene expression by the respective RC.

The invention is further directed to mammalian cells expressing several different genes of interests which are induced by one or more transcriptional switch(s) modulated by the presence of AT-rich DNA.

The invention is further directed to mammalian cells expressing several different genes of interests which are induced by two or more transcriptional switches which are all modulated by the presence of AT-rich DNA, preferably by the presence of the same linear AT-rich DNA fragment. The invention is further directed to cell based test systems using mammalian cells containing at least one transcriptional switch, preferably two different transcriptional switches, modulated by the presence of AT-rich DNA.

The following Figures and Examples are intended to illustrate various embodiments of the present invention. As such, the specific modifications discussed therein are not to be understood as limitations of the scope of the invention. It will be apparent to the person skilled in the art that various equivalents, changes, and modifications may be made without departing from the scope of the invention, and it is thus to be understood that such equivalent embodiments are to be included herein.

The project leading to this application has received funding from the European Union's Horizon 2020 research and innovation program under grant agreement No. 667079.

### Examples

### Example 1: The co-introduction of AT-DNA with an inducible EGFP and mCherry reporter plasmid results in a significantly better modulation of the reporter gene induction

One plasmid was constructed containing a bidirectional tetracycline inducible promotor capable of inducing the expression of the reporter gene enhanced green fluorescent protein (EGFP) as well as a suitable TF and a second plasmid containing a bidirectional tetracycline inducible promotor capable of inducing the expression of the red fluorescent protein mCherry. Both plasmids contained the same neomycin resistance gene. The OP consists of a bidirectional promotor containing two CMV minimal promotor sequences linked by seven tetracycline OS "tccctatcagtgatagaga" each spaced by the 18 base pair spacer "aacgtatgtcgagtttac". An EGFP reporter gene is fused downstream to one site of this bidirectional promotor and located downstream to the other site of the bidirectional promotor is a TF, consisting of a fusion of the Tn10-encoded Tet repressor protein and a Herpes simplex VP16 domain. This plasmid was co-transfected in CHO cells with the second plasmid containing the mCherry reporter gene fused to the tetracycline inducible promotor and a linear DNA containing the AT-DNA using FUGENE-HD (Promega®). The AT-DNA consists of the sequence of SEQ ID No. 1 (containing the sequence 1-3236 of Genbank: GM773871.1). All transfections were conducted according to the respective manufacturer's instructions. In order to generate stable transfected cell lines, cells were transfected at a confluence of 75 % in 12-well plates. After 48 hrs of cultivation, cells were selected and expanded under 0.6 mg/ml G418-sulfate (Invivogen®).

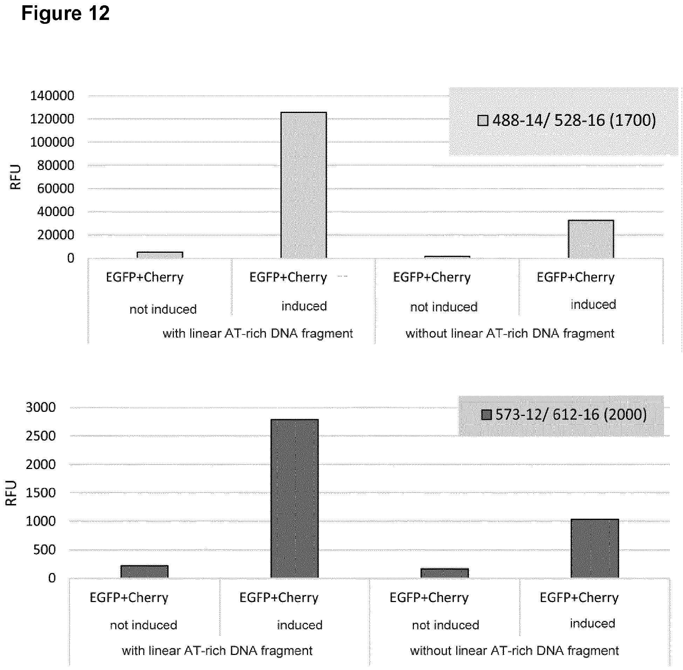

Surprisingly, the co-introduction of AT-DNA and the plasmids harboring the dox-off transcriptional switch controlling the inducible EGFP and the inducible mCherry expression resulted in a significant improvement of the yield of positive cell clones which survived the selection pressure. In addition, the co-introduction resulted in cell populations expressing their gene of interest in a stable well-defined amount and high expression level after induction. Both effects were unexpected because the expression of the TF is dependent on its transcription activator activity and cell does not continuously express any chimeric repressor or transactivator due to regulation of its expression by a constitutive promotor. Moreover, the AT-DNA was not part of the plasmid DNA comprising the dox-off transcriptional switch. 2 weeks after transfection cell populations which survived the selection process were characterized. After induction of EGFP and mCherry by withdrawing of doxycycline from the medium, EGFP and mCherry expression was characterized by fluorescence measurement in a microplate reader. As a control, the plasmids were transfected alone without AT-DNA. It has been found after induction that compared to the control the proportion of cells with an inducible EGFP and mCherry as well as the expression level of resistant cells was significantly increased (Figure 12).

### Example 2: Development of a Bicarbonate sensor cell line and an assay for measuring intracellular bicarbonate

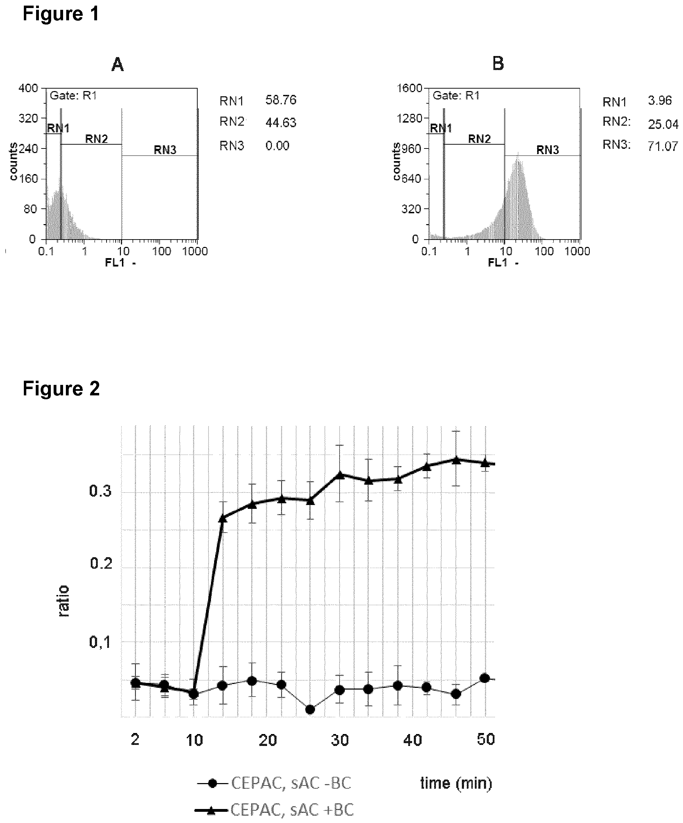

Two plasmids were constructed using the same bidirectional promotor as described in Example 1. Plasmid 1 carrying a neomycin resistance gene encoded sequences for the TF and a truncated adenylate cyclase type 10 isoform 1 (sAc,) (NCBI Reference Sequence: NP_060887.2 residues 1-469). Plasmid 2 carrying a hygromycin resistance gene encoded sequences for the TF and the cAMP sensor CEPAC (9). CHO cells were co-transfected with Plasmid 1, Plasmid 2 and with a linear DNA containing the AT-rich DNA using FUGENE-HD (Promega®). After 48 hrs of cultivation, cells were selected and expanded under 0.6 mg/ml G418-sulfate and 0.6 mg/ml hygromycin (Invivogen®). After 2 weeks, G418 and hygromycin resistant cells were induced by withdrawing of doxycycline from the medium. As control the plasmids were co-transfected without AT-DNA. It has been found after 24 hours of induction that compared to the control the resistant cells showed a morphology which was distinct from the control and expressed significantly more CEPAC. After induction an unexpectedly high proportion of cells (71%) showed a high CEPAC expression (Figure 1). After induction of sAc and CEPAC for 21h at 37°C and 5% CO₂ bicarbonate was depleted from the cell via replacement of culture medium with bicarbonate free buffer (126 mM NaCl, 5.4 mM KCI, 5.5 mM glucose, 0.8 mM MgCl₂, 25 mM HEPES, 1.8 mM CaCl₂, pH 6.6) for 3h at room temperature. Intracellular cAMP concentration was measured measuring the ratio of the emission (470 nm/535 nm) after excitation at 420 nm. High concentrated bicarbonate buffer (126 mM NaHCO₃, 5.4 mM KCI, 5.5 mM glucose, 0.8 mM MgCl₂, 25 mM HEPES, 1.8 mM CaCl₂, pH 7.7 replacement resulted in bicarbonate influx and concomitant activation of sAc which could be measured by an increase of the fluorescence ratio (Figure 2).

### Example 3: Development of a NSAID (pain killer pharmaceuticals) sensor cell line

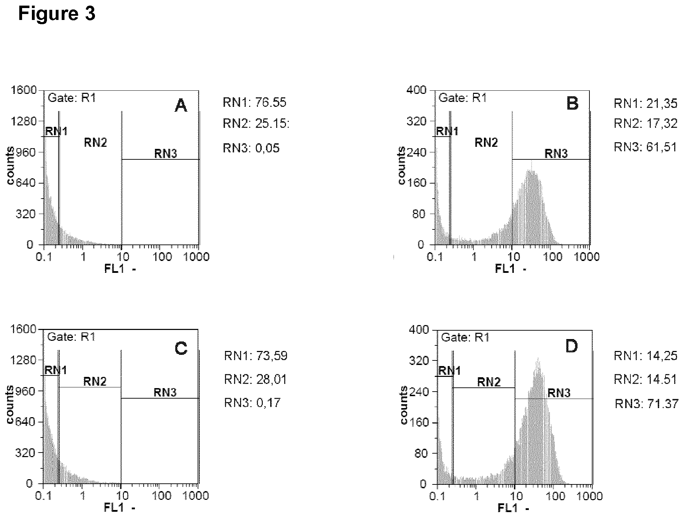

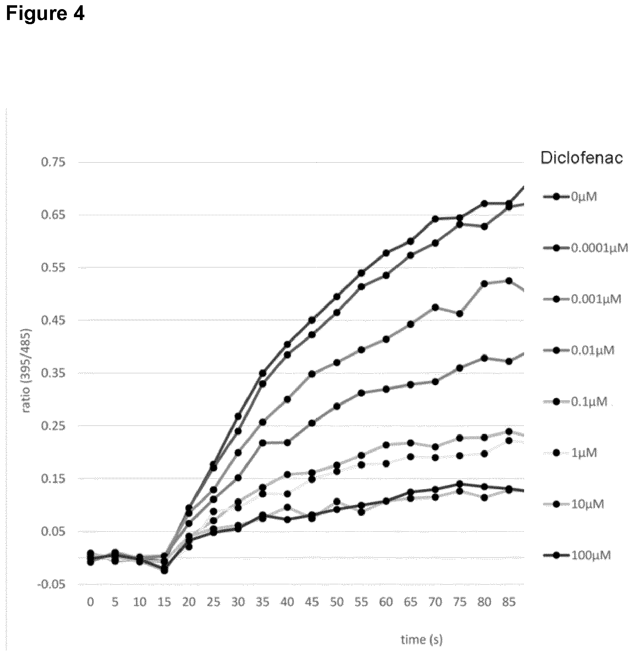

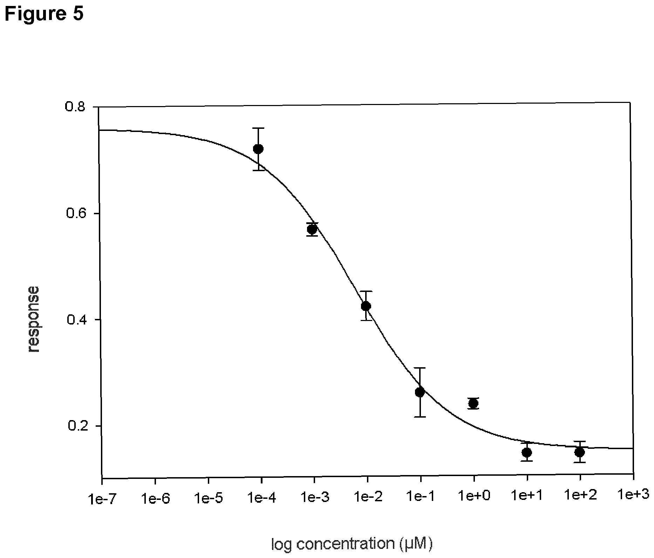

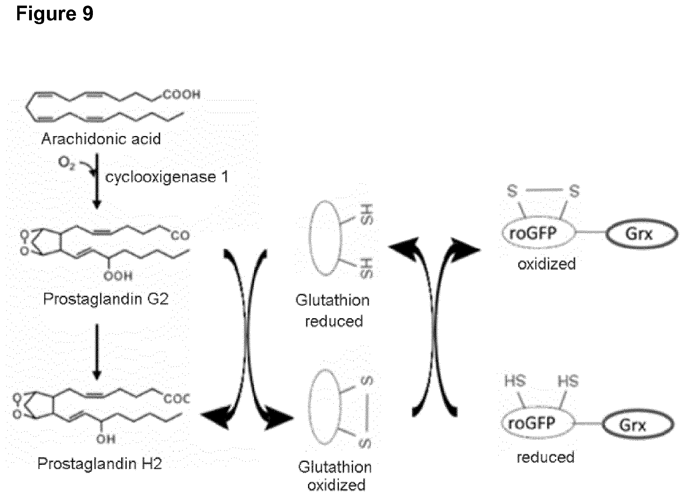

Two plasmids were constructed using the same bidirectional promotor as described in Example 1. Plasmid 1 carrying a neomycin resistance gene encoded sequences for the TF and human cyclooxygenase-1 (NCBI Reference Sequence: NP_000953.2). Plasmid 2 carrying a hygromycin resistance gene encoded sequences for the TF and the redox sensitive roGFP fused to glutarredoxin (grx-roGFP). CHO cells were co-transfected with Plasmid 1, Plasmid 2 and with a linear DNA containing the AT-DNA using FUGENE-HD (Promega®). After 48 h of cultivation, cells were selected and expanded under 0.6 mg/ml G418-sulfate and 0.6 mg/ml hygromycin (Invivogen). After 2 weeks, G418 and hygromycin resistant cells were induced by withdrawing of doxycycline from the medium. As control the plasmids were co-transfected without AT-DNA. It has been found after 24 hours of induction that compared to the control the proportion of cells with an inducible grx-roGFP as well as the grx-roGFP expression level of resistant cells was significantly increased. After the selection process grx-roGFP expression of two resistant cell populations were measured by FACS. After induction an unexpectedly high proportion of cells (60-70%) showed a high grx-roGFP expression (Figure 3). A cell based assay was developed in which the addition of the cyclooxygenase substrate arachidonic acid resulted in an increase of oxidized redox sensor which could be measured by measuring the fluorescence change (Figure 9). The oxidation of the redox sensor was dependent on the expression of cyclooxygenase, in a control cell expressing the redox sensor without simultaneous expression of cyclooxygenase-1 no oxidation of the redox sensor could be measured after addition of arachidonic acid. The preincubation of the sensor cell line with different amounts of non-steroidal anti-inflammatory drugs inhibited cyclooxygenase activity (Figure 4). With this assay, a concentration-response curve of diclofenac could be measured (Figure 5).

### Example 4: AT-DNA directed modulation of two gene switches (dox-off / coumermycin-on system)

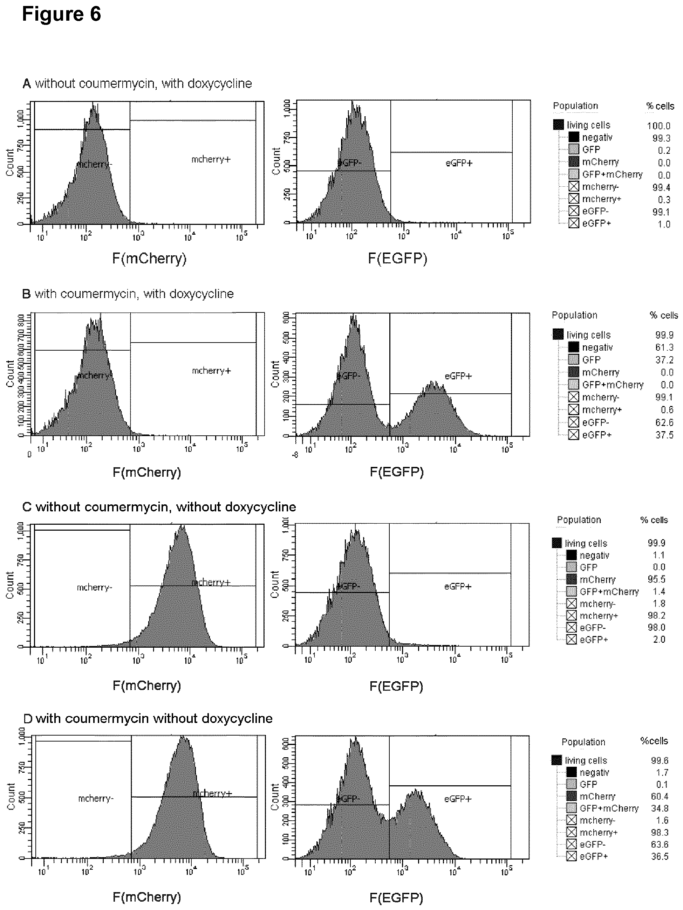

Two plasmids were constructed with different gene switches. Plasmid 1 carrying a neomycin resistence gene encoded sequences for the TF and the OP and the reporter gene mCherry (GenBank: AMO27233.1) as described in Example 1. This dox-off system gene switch induces mCherry expression after doxycycline withdrawal from the medium. Plasmid 2 carrying a hygromycin resistance gene encoded sequences for the coumermycin-on system TF under the control of a bidirectional promotor containing anappropriate OP for the coumermycin-on system. This TF comprise a N-terminal domain of the λ repressor (the cl gene product of bacteriophage λ) fused to the bacterial DNA gyrase B subunit followed by the transcription activation domain p65 NF-κB described in (5). The coumermycin-on system OP consists of a bidirectional promotor containing two CMV minimal promotor sequences linked by twelve OS "TACCTCTGGCGGTGATA" spaced by the 8 base pair linker "GTCGAGTT". Beside the TF, the bidirectional promotor was fused with the reporter gene EGFP. This coumermycin-on system gene switch induces EGFP expression after coumermycin addition to the medium. CHO cells were co-transfected with Plasmid 1, Plasmid 2 and with a linear DNA containing the AT-DNA using FUGENE-HD (Promega®). After 48 h of cultivation, cells were selected and expanded under 0.6 mg/ml G418-sulfate and 0.6 mg/ml hygromycin (Invivogen®). After 2 weeks, G418 and hygromycin resistant cells were induced. As control the plasmids were co-transfected without AT-DNA. After induction for 24h it has been found that in contrast to the control the major part of the resistant cells could be induced to express mCherry and/or EGFP (Figure 6). Only a minor part of resistant cells of the control showed an induction of mCherry after withdrawing of doxycycline from the medium and none of the resistant cells from the control could be induced to express mCherry and EGFP.

### Example 5: Development of a Cav1.2 cell line

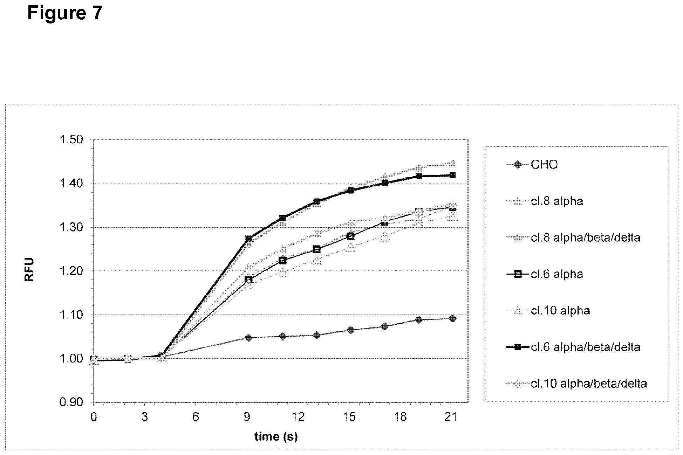

For the expression of a functional Cav1.2 ion channel three proteins, the pore forming unit and two associated proteins, have to be expressed at the same time. Therefore three plasmids were constructed. Plasmid 1 carrying the neomycin resistance gene encoded the human Cav1.2 alpha-1C pore forming unit (NCBI Reference Sequence: NP_000710.5) and the Dox-off system TF using the same bidirectional promotor as described in Example 1. Plasmid 2 carrying the Zeocin resistance gene encoded two comermycin-on system TF located downstream of both sites of the bidirectional promotor as described for plasmid 2 in Example 2. Plasmid 3 carrying a hygromycin resistance gene encoded the beta (beta2, NCBI Reference Sequence: NP_000715.2) and the delta (a2delta, NCBI Reference Sequence: NP_000713.2) Cav1.2 subunit proteins. Both genes were fused to the bidirectional promotor as described for plasmid 2 in Example 4. Plasmid 1 was co-transfected in CHO cells with AT-DNA as described in Example 1. After selection by 0.6 mg/ml G418 resistant clones were overtransfected with plasmid 2 and plasmid 3. After selection with Zeocin and Hygromycin resistant cells were subcloned and single cells were screened for ion channel activity. Three single Cav1.2 (alpha dox-off; beta/delta coumermycin-on) clones were characterized after 24 h induction of the pore forming unit alpha-1C alone and induction of the pore forming unit alpha-1C together with the associated subunits beta2 and a2delta. After induction of the pore forming unit alpha-1C alone a calcium influx could be measured which was clearly enhanced if the pore forming unit alpha-1C was induced together with the associated subunits beta2 and a2delta (Figure 7).

### Example 6: beta-blocker biosensor cell line.

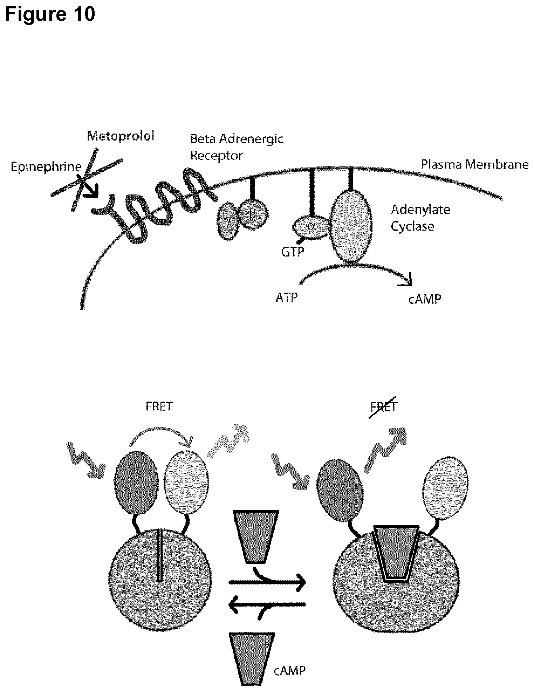

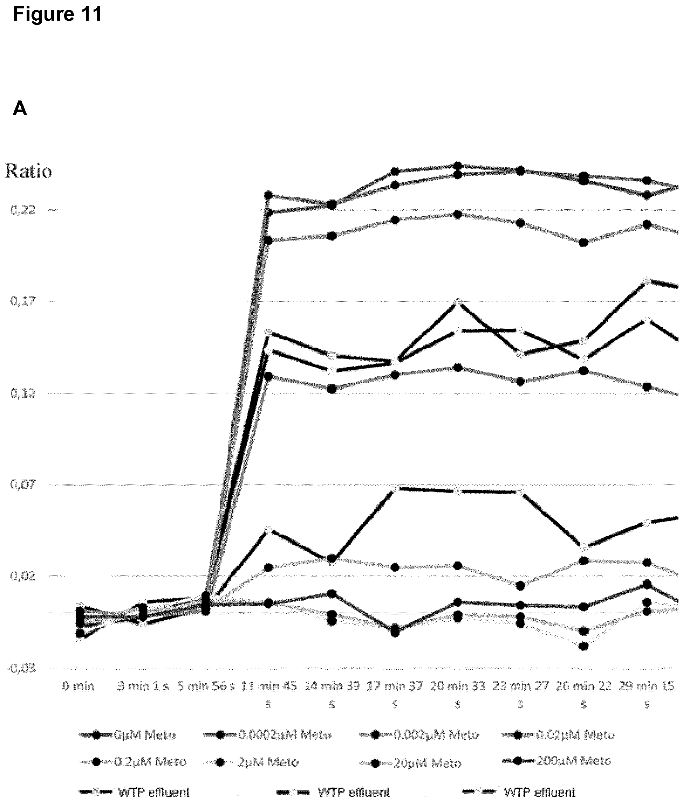

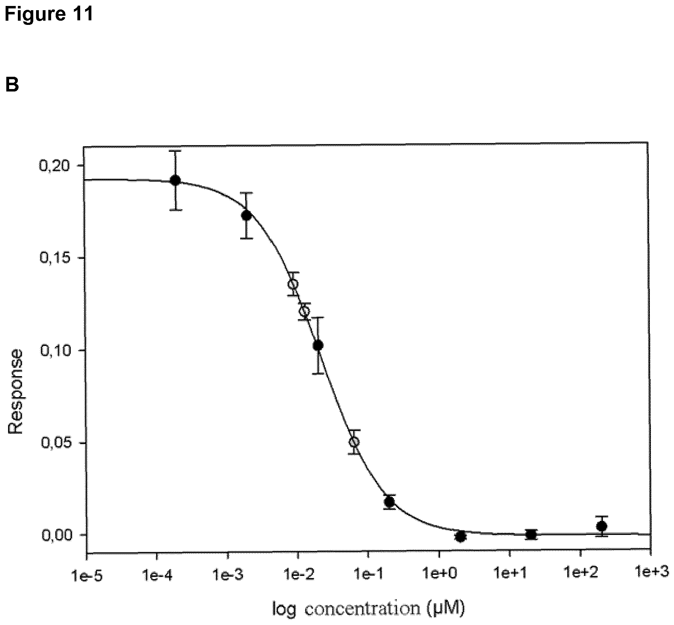

Two plasmids were constructed using the same bidirectional promotor as described in Example 1. Plasmid 1 carrying a neomycin resistance gene encoded sequences for the TF and human ß-adrenergic receptor (NCBI Reference Sequence: NP_000675.1). Plasmid 2 carrying a hygromycin resistance gene encoded sequences for the TF and the cAmp sensor CEPAC (9). CHO cells were co-transfected with Plasmid 1, Plasmid 2 and with a linear DNA containing the AT-DNA using FUGENE-HD (Promega®). After 48 h of cultivation, cells were selected and expanded under 0.6 mg/ml G418-sulfate and 0.6 mg/ml hygromycin (Invivogen®). Upon induction of transgene expression via doxycycline withdrawal from the culture medium, subclones were screened for ß1-adrenergic receptor activity using the agonist isoproterenol: upon receptor activation by addition of the agonist isoproterenol the intracellular cAMP level increases leading to an increase in the emission fluorescence ratio 470 nm/535 nm when excited at 420 nm (Figure 10). In the presence of beta-blocker, this ratio increase is reduced in a concentration-dependent fashion. The CEPAC-ß1-adrenergic receptor sensor cell line was used to assay for beta-blocker activities in wastewater samples (Figure 11A and 11B).

### Example 7: Development of a Calcium sensor cell line

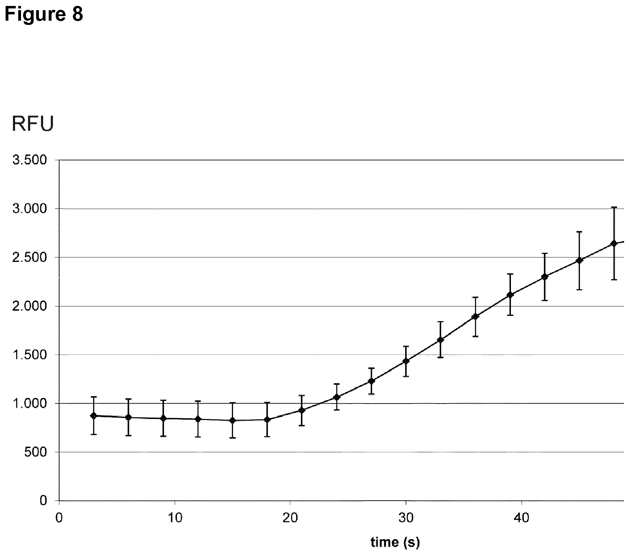

One plasmid was constructed using the same bidirectional promotor as described in Example 1. The plasmid carrying a neomycin resistance gene encoded sequences for the TF and the calcium sensor protein GCaMP3 (ADJ53338) (12). CHO cells were co-transfected with Plasmid and with a linear DNA containing the AT-DNA using FUGENE-HD (Promega®). After 48 h of cultivation, cells were selected and expanded under 0.6 mg/ml G418-sulfate and 0.6 mg/ml hygromycin (Invivogen®). After 2 weeks, G418 and hygromycin resistant cells were induced by withdrawing of doxycycline from the medium. (Figure 8)

### Brief Description of the Figures

**Figure 1****:** CHO cells were co-transfected with Plasmid 1 encoding the dox-off system TF and a truncated soluble adenylate cyclase and Plasmid 2 encoding the TF and cAMP sensor CEPAC whereby all genes were under the control of the dox-off gene switch and with a linear DNA containing the AT-DNA. After 2 weeks of selection with G418 and hygromycin, a resistant cell population was isolated. Without induction by withdrawing of doxycycline from the medium the resistant cell population does not show any significant CEPAC fluorescence **(A).** After 24 hours of induction, an unexpectedly high proportion of cells (71%) showed a high CEPAC expression **(B).**
**Figure 2****:** After induction of sAc and CEPAC, cells were incubated in bicarbonate free buffer (-BC). Intracellular cAMP concentration was measured measuring the ratio of the emission (470 nm/535 nm) after excitation at 420 nm. After 10 minutes +BC buffer was replaced by bicarbonate containing buffer (+BC). This resulted in activation of sAC and concomitant cAMP synthesis which was measured by an increase of the fluorescence ratio (Example 2).
**Figure 3****:** CHO cells were co-transfected with Plasmid 1 encoding the dox-off system TF and the human cyclooxygenase-1 and with Plasmid 2 encoding sequences for the TF and the redox sensitive roGFP fused to glutarredoxin (grx-roGFP) whereby all genes were under the control of the dox-off gene switch and with a linear DNA containing the AT-DNA. After 2 weeks of selection with G418 and hygromycin two resistant cell populations were isolated. Expression of the grx-roGFP expression of the two resistant cell populations were measured without **(A** and **C)** and with induction **(B** and **D)** by FACS. After induction, an unexpectedly high proportion of cells (61-71%) showed a high grx-roGFP expression level.
**Figure 4****:** The resistant cell population expressing the grx-roGFP together with the cyclooxygenase was used for monitoring activity of inhibitors of cyclooxygenase. After adding of the cyclooxygenase substrate arachidonic acid oxidized redox sensor generated by oxidized glutathione was determined by measuring the increase in the excitation fluorescence ratio 395 nm/485 nm, emitted at 528 nm. The signal correlates with activity of cyclooxygenase and is inhibited in a concentration dependent manner by the inhibitor diclofenac.
**Figure 5****:** Concentration-response curve of diclofenac. The resistant cell population expressing the grx-roGFP together with the cyclooxygenase was used for measuring a concentration-response curve of the non-steroidal anti-inflammatory drug diclofenac. IC50 value of 6 nM was calculated by fitting of the concentration response curve (sigmoidal fitting, 4 parameter, SigmaPlot).
**Figure 6****:** CHO cells harboring the tet-off gene switch for the inducible expression of mCherry and the coumermycin-on gene switch for the expression of EGFP respectively co-transfected with the modulating AT-rich DNA element were analyzed by FACS. Without coumermycin and with doxycycline no expression of mCherry or EGFP was measured (A). Induction of the coumermycin-on switch controlled expression of EGFP was measured for 38% of the resistant cell population due to addition of coumermycin and doxycycline to the medium **(B).** Induction of the dox-off switch controlled expression of mCherry was measured for 98% of the resistant cell population due to withdrawing of doxycycline from the medium **(C).** Induction of the coumermycin-on switch controlled expression of EGFP and at the same time the induction of the dox-off system controlled expression of mCherry was achieved by adding coumermycin to the medium and withdrawing of doxycycline from the medium. Here 37% of the resistant cell population expressed mCherry and EGFP both at high expression levels **(D).**
**Figure 7****:** CHO cells harboring the tet-off system switch for the inducible expression of Cav1.2 alpha-1C pore forming unit and the coumermycin-on system switch for the expression of the beta (beta2, NCBI Reference Sequence: NP_000715.2) and the delta (a2delta, NCBI Reference Sequence: NP_000713.2) Cav1.2 subunit proteins, respectively, co-transfected with the modulating AT-rich DNA element were analyzed by a calcium influx assay. After induction of the pore forming unit alpha-1C alone by withdrawing of doxycycline from the medium a calcium influx could be measured which was clearly enhanced if the pore forming unit alpha-1C was induced together with the associated subunits beta2 and a2delta due to adding of coumermycin to the medium and withdrawing of doxycycline from the medium.
**Figure 8****:** After induction of GCaMP3, fluorescence of GCaMP3 was measured. After 20 seconds, ionomycin containing buffer was added. Calcium dependent fluorescence change was measured in relative fluorescence units (RFU) (Example 7).
**Figure 9****:** The NSAID biosensor cell line expresses after induction cyclooxygenase 1 and the redox sensitive GFP fused to glutaredoxine (roGFP-Grx). The addition of the cyclooxygenase substrate arachidonic acid resulted in an increase of reactive oxygen species and oxidized redox sensor which could be measured by measuring the fluorescence change of the redoxsensitive roGFP.
**Figure 10****:** Upon ß-adrenergic receptor activation the intracellular cAMP level increases leading to an increase in the emission fluorescence ratio 470 nm/535 nm of the cAMP sensor CEPAC when excited at 420 nm.
**Figure 11****:** Response curves of the beta-blocker metoprolol and SPE-enriched wastewater samples, each at different concentrations, are shown **(A).** Concentration-response curve was generated from metoprolol standard at different concentrations **(B).** From this grading curve beta-blocker activity in solid phase enriched wastewater sample was determined at different concentrations.
**Figure 12****:** G418 resistant CHO cell populations resulting after co-transfection of two plasmids with the linear AT-rich DNA fragment and without the linear AT-rich DNA fragment and selection with G418 were cultivated in 96 well plates up to confluency. Induction of EGFP and mCherry followed after doxycycline withdraw from the medium. It has been found after induction that the proportion of cells with an inducible EGFP and mCherry as well as the expression level of resistant cells was significantly increased compared to the control were the two plasmids were co-transfected without the linear AT-rich DNA fragment.

### References

1. Girod, P.A., M. Zahn-Zabal, and N. Mermod, Use of the chicken lysozyme 5' matrix attachment region to generate high producer CHO cell lines. Biotechnol Bioeng, 2005. 91(1): p. 1-11.
2. Zahn-Zabal, M., et al., Development of stable cell lines for production or regulated expression using matrix attachment regions. J Biotechnol, 2001. 87(1): p. 29-42.
3. Imhof, M.O., P. Chatellard, and N. Mermod, A regulatory network for the efficient control of trans gene expression. J Gene Med, 2000. 2(2): p. 107-16.
4. Gossen, M., et al., Transcriptional activation by tetracyclines in mammalian cells. Science, 1995. 268(5218): p. 1766-9.
5. Zhao, H.F., et al., A coumermycinlnovobiocin-regulated gene expression system. Hum Gene Ther, 2003. 14(17): p. 1619-29.
6. Auslander, S. and M. Fussenegger, From gene switches to mammalian designer cells: present and future prospects. Trends Biotechnol, 2013. 31 (3): p. 155-68.
7. Roscilli, G., et al., Long-term and tight control of gene expression in mouse skeletal muscle by a new hybrid human transcription factor. Mol Ther, 2002. 6(5): p. 653-63.
8. Weber, W., et al., Vitamin H-regulated transgene expression in mammalian cells. Nucleic Acids Res, 2007. 35(17): p. e116.
9. Wissmann, A., et al., Tn10 tet operator mutations affecting Tet repressor recognition. Nucleic Acids Res, 1986. 14(10): p. 4253-66.
10. Romig, H., et al., Characterisation of two intronic nuclear-matrix-attachment regions in the human DNA topoisomerase I gene. Eur J Biochem, 1994. 221 (1): p. 411-9.
11. Romig, H., GeneBank L23999.1, 1995.
12. Tian, L., et al., Imaging neural activity in worms, flies and mice with improved GCaMP calcium indicators. Nat Methods, 2009. 6(12): p. 875-81.

## Claims

1. A method for providing a cell line expressing at least two different recombinant proteins, comprising the steps of
a) providing mammalian cells in cell culture;
b) providing at least one plasmid, wherein the at least one plasmid contains a DNA sequence encoding for a transcriptional activator under the control of at least one promotor region operably linked to at least one operator site (OP), wherein the operator site (OP) is responsive for binding the transcription factor domain of the transcriptional activator;
c) transfecting the mammalian cells with at least one linear DNA fragment comprising or consisting of an AT-rich nucleotide sequence and, in parallel, with the at least one plasmid of step b), **characterized in that** the plasmid of step b) further contains a DNA sequence encoding for at least one recombinant protein of interest, wherein the expression of the at least one recombinant protein of interest is under the control of the same gene switch as the transcriptional activator;
d) inducing the concomitant self-amplification of the transcriptional activator and the expression of the at least one recombinant protein of interest by incubating the mammalian cells under conditions of a transcriptional switch;
e) monitoring the level of expression of the at least one recombinant protein of interest by means of visual inspection or by means of a functional readout.

2. The method of claim 1, wherein the visual inspection in step e) is by means of fluorescent readout, preferably by means of quantitative fluorescent readout.

3. The method of any one of claim 1 or 2, wherein the concomitant self-amplification of the transcriptional activator and the expression of the at least one recombinant protein of interest in step d) is induced by incubating the mammalians cells under conditions of the same transcriptional switch.

4. The method of any one of claims 1 to 3, where the inducible concomitant self-amplification of the transcriptional activator and the expression of the at least one recombinant protein of interest in step d) involves incubating of the mammalian cells under conditions of the dox-off system, wherein the dox-off system is **characterized by** the interaction of the Tn10-encoded Tet repressor protein (TetR) and a corresponding operator site (OP).

5. The method of any one of claims 1 to 3, where the inducible concomitant self-amplification of the transcriptional activator and the expression of the at least one recombinant protein of interest in step d) involves incubating of the mammalian cells under conditions of the coumermycin-on system, wherein the coumermycin-on system is **characterized by** the interaction of a fusion protein composed of the N-terminal domain of the λ repressor fused to the bacterial DNA gyrase B subunit followed by the transcription activation domain p65 NF-κB and a corresponding operator site (OP).

6. The method of any one of claims 1 to 5, wherein the at least one linear DNA fragment comprising or consisting of an AT-rich nucleotide sequence has a length of between 3500 and 2700 nucleotides, preferably a length of between 3300 and 2800 nucleotides, more preferably a length of between 3280 and 2900 nucleotides.

7. The method of any one of claims 1 to 6, wherein the at least one linear DNA fragment comprising or consisting of an AT-rich nucleotide sequences comprises or is composed of a sequence corresponding to the AT rich MII region of intron 13 of human topoisomerase I.

8. The method of any one of claims 1 to 7, wherein the at least one linear DNA fragment comprising or consisting of an AT-rich nucleotide sequence has the sequence of SEQ ID No. 1, SEQ ID No. 2 or SEQ ID No. 3, or any combination thereof.

9. The method of any one of claims 1 to 8, wherein the at least one linear DNA fragment comprising or consisting of an AT-rich nucleotide sequence is co-transfected with the at least one plasmid in step c) at a molar ratio of between 0.1 (AT-rich DNA fragment) to 8 (plasmid), preferably at a molar ratio of between 0.2 (AT-rich DNA fragment) to 6 (plasmid), more preferably at a molar ratio of between 0.5 (AT-rich DNA fragment) to 4 (plasmid).

10. A method for providing a cell based assay for analyzing the function of a protein complex of interest, comprising the steps of
a) providing mammalian cells in cell culture, wherein the cells are derived from a cell line obtained by the method of claim 1; and
b) subjecting the mammalian cells to conditions of a cell assay, wherein the cell assay is **characterized by**
1) measuring the intracellular concentration of bicarbonate; or
2) measuring the intracellular concentration of calcium; or
3) measuring the activity of ion channels; or
4) measuring the activity of the ion channel Cav1.2; or
5) measuring intracellular cyclooxygenase activity; or
6) measuring the activity of G-protein coupled receptors (GPCRs); or
7) measuring the activity and/or inhibition of the G-protein coupled beta-adrenergic receptors.

11. A mammalian cell line, wherein the mammalian cell line is **characterized by**
i) the presence of a transcription factor (TF), wherein the transcription factor binds to an operator site (OP) containing promotor sequence in response to the presence of at least one compound which is to be added to the cell culture medium, or, alternatively, in response to the absence of at least one compound which is to be removed from the cell culture medium;
ii) the presence of a promotor sequence, or a fragment thereof, operatively linked to the operator site (OP), wherein the operator site is specific for binding of the transcription factor (TF);
iii) the presence of a DNA fragment comprising or consisting of an AT-rich nucleotide sequence, wherein the DNA fragment results from of the integration of a linear DNA fragment.

12. The mammalian cell line of claim 11, wherein the DNA fragment comprising or consisting of an AT-rich nucleotide sequence is capable of allowing a self-amplification of the transcriptional activator without the need of a constitutively expressed chimeric repressor or transactivator.

13. The mammalian cell line of claim 11 or 12, wherein the DNA fragment comprising or consisting of an AT-rich nucleotide sequence is capable of modulating the interaction between the transcriptional activator and the promotor sequence, preferably of modulating the interaction with the operator site, or is capable of enhancing the expression of a co-transfected gene of interest.

14. The mammalian cell line of any one of claims 11 to 13, wherein the DNA fragment comprising or consisting of an AT-rich nucleotide sequence corresponds to or is identical to the sequence of the AT-rich MII region of intron 13 of human topoisomerase I.

15. The mammalian cell line of any one of claims 11 to 14, wherein the DNA fragment comprising or consisting of an AT-rich nucleotide sequences has the sequence of SEQ ID No. 1, SEQ ID No. 2 or SEQ ID No. 3, or any combination thereof.

16. The mammalian cell line of any one of claims 11 to 15, wherein the cell line stably expresses
a) at least two recombinant proteins, wherein the at least two recombinant proteins enable the measurement of intracellular bicarbonate concentration changes;
b) at least two recombinant proteins, wherein the at least two recombinant proteins enable the measurement of intracellular calcium concentration changes;
c) at least two recombinant proteins, wherein the at least two recombinant proteins enable the measurement of ion channel activity;
d) at least two recombinant proteins, wherein the at least two recombinant proteins enable the measurement of the Cav1.2 ion channel activity;
e) cyclooxygenase and a redox sensor protein, wherein the cyclooxygenase and the redox sensor protein enable the measurement of cyclooxygenase activity;
f) at least two recombinant proteins, wherein the at least two recombinant proteins enable the measurement of G-protein coupled receptor (GPCRs) activity;
g) at least two recombinant proteins, wherein the at least two recombinant proteins enable the measurement of beta adrenergic receptor activity.


SEQUENCE LISTING

<110>  Steinbeis Innovation gGmbH

<120>  Inducible gene expression by linear AT-rich DNA fragments in
       mammalian cells and methods relating thereto

<130>  S70516EP1

<160>  6

<170>  PatentIn version 3.5

<210>  1
<211>  3272
<212>  DNA
<213>  Artificial Sequence

<220>
<223>  containing the sequence 1-3236 of GenBank: GM773871.1

<400>  1
gtcgactcta gaggatccca ataggagtca ttaaaggcct ggaaaagtgg tgccattagg      60

agaaaaagaa atgatttctt gagcttgctc tcagttctct tttaggctgt cttgtactca     120

gcagaatagt gagatcttca aaggttgggg tttgatagtg ccttgaataa tttttaactt     180

tatattgcca gcggaagaag cattctcttt ttagatttaa aaaatgtaga tacaaatatt     240

aggggtttta tttttagtga aacatttcaa acatacagga atagataatt atgtaatgaa     300

cactcgtatg tccaccatct ggctttgtaa aatcttaaaa ttatgtctta tgtgctcaat     360

tgttttattt cataaaagat actgataaac atagctgaag tcacttgtat accattcacc     420

ttcttccctg tagattacta tgaactcggt ctttttattc tcatacatat tttttgtatt     480

tttgcagtat atttatgtgt tcataaacaa tatgtaattt tacaatatgt aacacactag     540

taacatacta atttaaaact tgtttttagt ttacaatatg ttgtaaacta ttgtaagcta     600

aagacatatt gtacaaccta ttgttaaata aaaacaggtt ttagttttaa attaggtatg     660

ttactgggat cattctgcaa cttgtttatt cctctccagc tttgattttg tggttttatt     720

atcttaacct acacttttaa ttaatccatt ttatttgtta catggtattc tattatatca     780

taaaaactta tctattctgt tggtttttgt tgttggtcat ttgagaccat gtcttcgctc     840

tgtcacccag gctggagtac agtggcgtga tcttggctca ctgtgacctc tgcctcccgg     900

attcaagtgg ttttggtgcc tcagcctcct gagtagctgg gattataggc gtgtgccacc     960

atgcccagct aatttttgta tttttaatag agacgggatt tcaccatgtt ggccaggctg    1020

gtcttgaact gacctcaagt gatctgctca cctcagctgc acaaagtgct gggattacag    1080

gtgttagcca accaataccc tgcctctatt ctcttgttaa gaggcattta gcatggttaa    1140

tacagtctct tgccctcata aacagtgctg ggaaggaaac acatgttctt gtgtatattg    1200

aatgaaattt gtttatacat tagatatttc caaatgttct ctttaagtac ttcagtttac    1260

atcattactc tcctcctccc tcccctccca cccccaccca caacagtatt cctctttttc 1320

catatccttg ctaatgtttc aaagttttgc tttttacatt tgggtcttag atccactaga 1380

atgtattttt gcattgggat gaagttgaaa cctaatatat tttccaaatg agtaaactgt 1440

tgtcacagaa ctatttagtt gtattacctc ctctcttgta tatcagatat atctacatat 1500

atgtcagact gtttctgggc tgtctgtcct ctttaattag ttcgtgtatc tgtttctgca 1560

tcagtagcat actgtcttaa ctactgtagc tttataaagt ctattgagta ggacaagttt 1620

gtttcattct tcaaaattgc tttggctatt cttggccctc tgctgtttca tattaacttt 1680

cagataaact tgtcaaattc taatgaaaac tgttgataaa cttgttgatt aacaaattct 1740

aataaaaact gttgagattt ttattggaat tgcaatacat ttatagatta acggagaaag 1800

atattgacaa tacaattgag tttccaattc acgaacatgt tatacctctc cattaattca 1860

tgtcttttga atgtatccac caatatggtt ttgtaatttt cttcataaag gacatttaaa 1920

attcttattt taagtgatct tatagtttta tgctaacgtg aatgagattt ttccattatg 1980

tttctgttgg ttattcctga agtggtaatg cttataattt tggggtgttg gtcttgtatc 2040

tgcctaggag ctcgatttta gctcgatttt agctcgattt tagctcgatt ttagctcgat 2100

tttagctcga ttttagctcg attttagctc gattttagct cgattttagc tcgattttag 2160

ctcgatttta gctcgatttt agctcgattt tagctcgatt ttagctcgat tttagctcga 2220

ttttagctcg attttagctc gattttagct cgattttagc tcgattttag ctcgatttta 2280

gctcgatttt agctcgactc gagcctagga gatacaagag gtgctcagag ttgttcaggg 2340

ttgctgaact cttagttcta aaagtgtctg tcatttgggg tttctatgta gataatttaa 2400

ttatctataa aaacagttct tcattttcag ttcatatatt tcatattttc ttaagtttta 2460

atttttattt ttaaacacaa ttatccataa accctaaccc tttccctagt caacagcagt 2520

cacagccaaa tgttttatta attgctatac tcagtgtttc ttgtatctca taccttctgg 2580

ggtttcttgt cttgttgaaa tacacccttt aatgtttctt tagtgaagac ccaacagtgg 2640

cactcactca cctttgttta cctgaaaatt tctttatttt catcttaatt catagtctgt 2700

cttttctcca gtcaaggaag tgtcttatag ggaagattct ggtttcacta tcgtgtatcc 2760

aggatatata tgtatttata gatagacttt taatctgagg actaatgtat tttatcctac 2820

agtattacca atcattattt cttccataac ttctagacca ttccttttgt acttcttttt 2880

tagagtcctt attagatgag tgttgactct tttcaatcta gacatctttt ttaaactata 2940

ttttcatact ctttgtctct ttaggtctga ttttttaagt tcaggggata tttcattttg 3000

ggtgagttgt agcactactt caattcacta attctaatta tatttaatct acaagttatt 3060

ccatctataa tttatttcaa ttaccacttt ttgttttcaa aatttctaat tttatatctg 3120

attttgtttc atttttgttt tataatttca tgttctttct agattttaca tctttttatg 3180

catactaaac atactcactt gaaagtcttt gtaagattgt tctataaaat gttacctgaa 3240

gtgaattcac gcgtggtacc tctagagtcg ac 3272

<210> 2
<211> 2974
<212> DNA
<213> Homo sapiens

<400> 2
ggatcccaat aggagtcatt aaaggcctgg aaaagtggtg ccattaggag aaaaagaaat 60

gatttcttga gcttgctctc agttctcttt taggctgtct tgtactcagc agaatagtga 120

gatcttcaaa ggttggggtt tgatagtgcc ttgaataatt tttaacttta tattgccagc 180

ggaagaagca ttctcttttt agatttaaaa aatgtagata caaatattag gggttttatt 240

tttagtgaaa catttcaaac atacaggaat agataattat gtaatgaaca ctcgtatgtc 300

caccatctgg ctttgtaaaa tcttaaaatt atgtcttatg tgctcaattg ttttatttca 360

taaaagatac tgataaacat agctgaagtc acttgtatac cattcacctt cttccctgta 420

gattactatg aactcggtct ttttattctc atacatattt tttgtatttt tgcagtatat 480

ttatgtgttc ataaacaata tgtaatttta caatatgtaa cacactagta acatactaat 540

ttaaaacttg tttttagttt acaatatgtt gtaaactatt gtaagctaaa gacatattgt 600

acaacctatt gttaaataaa aacaggtttt agttttaaat taggtatgtt actgggatca 660

ttctgcaact tgtttattcc tctccagctt tgattttgtg gttttattat cttaacctac 720

acttttaatt aatccatttt atttgttaca tggtattcta ttatatcata aaaacttatc 780

tattctgttg gtttttgttg ttggtcattt gagaccatgt cttcgctctg tcacccaggc 840

tggagtacag tggcgtgatc ttggctcact gtgacctctg cctcccggat tcaagtggtt 900

ttggtgcctc agcctcctga gtagctggga ttataggcgt gtgccaccat gcccagctaa 960

tttttgtatt tttaatagag acgggatttc accatgttgg ccaggctggt cttgaactga 1020

cctcaagtga tctgctcacc tcagctgcac aaagtgctgg gattacaggt gttagccaac 1080

caataccctg cctctattct cttgttaaga ggcatttagc atggttaata cagtctcttg 1140

ccctcataaa cagtgctggg aaggaaacac atgttcttgt gtatattgaa tgaaatttgt 1200

ttatacatta gatatttcca aatgttctct ttaagtactt cagtttacat cattactctc 1260

ctcctccctc ccctcccacc cccacccaca acagtattcc tctttttcca tatccttgct 1320

aatgtttcaa agttttgctt tttacatttg ggtcttagat ccactagaat gtatttttgc 1380

attgggatga agttgaaacc taatatattt tccaaatgag taaactgttg tcacagaact 1440

atttagttgt attacctcct ctcttgtata tcagatatat ctacatatat gtcagactgt 1500

ttctgggctg tctgtcctct ttaattagtt cgtgtatctg tttctgcatc agtagcatac 1560

tgtcttaact actgtagctt tataaagtct attgagtagg acaagtttgt ttcattcttc 1620

aaaattgctt tggctattct tggccctctg ctgtttcata ttaactttca gataaacttg 1680

tcaaattcta atgaaaactg ttgataaact tgttgattaa caaattctaa taaaaactgt 1740

tgagattttt attggaattg caatacattt atagattaac ggagaaagat attgacaata 1800

caattgagtt tccaattcac gaacatgtta tacctctcca ttaattcatg tcttttgaat 1860

gtatccacca atatggtttt gtaattttct tcataaagga catttaaaat tcttatttta 1920

agtgatctta tagttttatg ctaacgtgaa tgagattttt ccattatgtt tctgttggtt 1980

attcctgaag tggtaatgct tataattttg gggtgttggt cttgtatctg gcagcagata 2040

caagaggtgc tcagagttgt tcagggttgc tgaactctta gttctaaaag tgtctgtcat 2100

ttggggtttc tatgtagata atttaattat ctataaaaac agttcttcat tttcagttca 2160

tatatttcat attttcttaa gttttaattt ttatttttaa acacaattat ccataaaccc 2220

taaccctttc cctagtcaac agcagtcaca gccaaatgtt ttattaattg ctatactcag 2280

tgtttcttgt atctcatacc ttctggggtt tcttgtcttg ttgaaataca ccctttaatg 2340

tttctttagt gaagacccaa cagtggcact cactcacctt tgtttacctg aaaatttctt 2400

tattttcatc ttaattcata gtctgtcttt tctccagtca aggaagtgtc ttatagggaa 2460

gattctggtt tcactatcgt gtatccagga tatatatgta tttatagata gacttttaat 2520

ctgaggacta atgtatttta tcctacagta ttaccaatca ttatttcttc cataacttct 2580

agaccattcc ttttgtactt cttttttaga gtccttatta gatgagtgtt gactcttttc 2640

aatctagaca tcttttttaa actatatttt catactcttt gtctctttag gtctgatttt 2700

ttaagttcag gggatatttc attttgggtg agttgtagca ctacttcaat tcactaattc 2760

taattatatt taatctacaa gttattccat ctataattta tttcaattac cactttttgt 2820

tttcaaaatt tctaatttta tatctgattt tgtttcattt ttgttttata atttcatgtt 2880

ctttctagat tttacatctt tttatgcata ctaaacatac tcacttgaaa gtctttgtaa 2940

gattgttcta taaaatgtta cctgaagtga attc 2974

<210> 3
<211> 2987
<212> DNA
<213> Homo sapiens

<400> 3
ggatcccaat aggagtcatt aaaggcctgg aaaagtggtg ccattaggag aaaaagaaat 60

gatttcttga gcttgctctc agttctcttt taggctgtct tgtactcagc agaatagtga 120

gatcttcaaa ggttggggtt tgatagtgcc ttgaataatt tttaacttta tattgccagc 180

ggaagaagca ttctcttttt agatttaaaa aatgtagata caaatattag gggtttattt 240

tttagtgaaa catttcaaac atacaggaat agataattat gtaatgaaca ctcgtatgtc 300

caccatctgg ctttgtaaaa tcttaaaatt atgtcttatg tgctcaattg ttttatttca 360

taaaagatac tgataaacat agctgaagtc acttgtatac cattcacctt cttccctgta 420

gattactatg aactcggtct ttttattctc atacatattt tttgtatttt tgcagtatat 480

ttatgtgttc ataaacaata tgtaatttta caatatgtaa cacactagta acatactaat 540

ttaaaacttg tttttagttt acaatatgtt gtaaactatt gtaagctaaa gacatattgt 600

acaacctatt gtaaaataaa aacaggtttt agttttaaat taggtatgtt actgggatca 660

ttctgcaact tgtttattcc tctccagctt tgattttgtg gttttattat cttaacctac 720

acttttaatt aatccatttt atttgttaca tggtattcta ttatatcata aaaacttatc 780

tattctgttg gtttttgttg ttggtcattt gagaccatgt cttcgctctg tcacccaggc 840

tggagtacag tggcgtgatc ttggctcact gtgacctctg cctcccggat tcaagtggtt 900

ttggtgcctc agcctcctga gtagctggga ttataggcgt gtgccaccat gcccagctaa 960

tttttgtatt tttaatagag acgggatttc accatgttgg ccaggctggt cttgaactga 1020

cctcaagtga tctgctcacc tcagctgcac aaagtgctgg gattacaggt gttagccacc 1080

ataccctgcc tctattctct tgttaagagg catttagcat ggttatacag tctcttgccc 1140

tcataaacag tgctggaaga aacacatgtt tcttgtgtat atgaatgaaa atttgtttta 1200

tacattagat atttccaaat tgttctctta agtacttcag tttacatcat tactctcctc 1260

ctccctcccc tcccaccccc acccacaaca gtattcctct ttttccatat ccttgctaat 1320

gtttctaaag ttttgctttt tacatttggg tcttagatcc actagaatgt atttttgcat 1380

tgggatgaag ttgaaaccta atatattttc caaatgagta aactgttgtc acagaactat 1440

ttagttgtat tacctcctct cttgtatatc agatatatct acatatatgt cagactgttt 1500

ctgggctgtc tgtcctcttt aattagttcg tgtatctgtt tctgcatcag tagcatactg 1560

tcttaactac tgtagcttta taaagtctat tgagtaggac aagtttgttt cattcttcaa 1620

aattgctttg gctattcttg gccctctgct gtttcatatt aactttcaga taaacttgtc 1680

aaattctaat gaaaactgtt gataaacttg ttgattaaca aattctaata aaaactgttg 1740

agatttttat tggaattgca atacatttat agattaacgg agaaagatat tgacaataca 1800

attgagtttc caattcacga acatgttata cctctccatt aattcatgtc ttttgaatgt 1860

atccaccaat atggttttgt aattttcttc ataaaggttt tacatttaaa aaattcttat 1920

ttttaagtga tcttatagtt tttattgcta atgtgaatga gatttttttc cattatgttt 1980

ctgttggtta ttcctgaagt ggtaatgctt ataattttgg ggtgttggtc ttgtatctgg 2040

cagcagatac aagaggtgct cagagttgtt cagggttgct gaactcttag ttctaaaagt 2100

gtctgtcatt tggggtttct atgtagataa tttaattatc tataaaaaca gttcttcatt 2160

ttcagttcat atatttcata tttctttaag ttttaatttt tatttttaaa cacaattatc 2220

cataaaaccc taaccctttc cctagtcaac agcagtcaca gccaaatgtt ttattaattg 2280

ctatactcag tgtttcttgt atctcatacc ttctggggtt tcttgtcttg ttgaaataca 2340

ccctttaatg tttctttagt gaagacccaa cagtggcact cactcacctt tgtttacctg 2400

aaaatttctt tattttcatc ttaattcata gtctgtcttt tctccagtca aggaagtgtc 2460

ttatagggaa gattctggtt tcactatgct gtatccaggg atatatatgt atttatagat 2520

agacttttaa tctgaggact aatgtatttt atcctacagt attaccaatc attatttctt 2580

ccataacttc tagaccattc cttttgtact tcttttttag agtcctatta gatgagtgtt 2640

gacacttttc aatctagaca tcttttttaa actatatttt catactcttt gtctctttag 2700

gtctgatttt ttaagttcag gggaatattt cattttgggt gagttgtagc actcacttcc 2760

aattcactaa ttctaattat atttaatcta caagttattc catctataat ttatttcaat 2820

taccactttt tgttttcaaa atttctaatt ttatatctga ttttgtttca tttttgtttt 2880

ataatttcat gttctttcta gattttacat ctttttatgc atactaaaca tactcacttg 2940

aaagtctttg taagattgtt ctataaaatg ttacctgaag tgaattc 2987

<210> 4
<211> 19
<212> DNA
<213> Artificial Sequence

<220>
<223> Tetracycline operator sequence

<400> 4
tccctatcag tgatagaga 19

<210> 5
<211> 18
<212> DNA
<213> Artificial Sequence

<220>
<223> Spacer

<400> 5
aacgtatgtc gagtttac 18

<210> 6
<211> 17
<212> DNA
<213> Artificial Sequence

<220>
<223> Operator sequence

<400>  6
tacctctggc ggtgata                                                    17